(19) Europäisches Patentamt
European Patent Office
Office européen des brevets

(11) Veröffentlichungsnummer: **0 242 687**
**A2**

# (12) EUROPÄISCHE PATENTANMELDUNG

(21) Anmeldenummer: **87105059.7**

(22) Anmeldetag: **06.04.87**

(51) Int. Cl.[4]: **C07D 231/38** , C07D 401/04 , //C07C109/04,C07D231/40,A01-N43/56

(30) Priorität: **17.04.86 DE 3612940**

(43) Veröffentlichungstag der Anmeldung:
**28.10.87 Patentblatt 87/44**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gallenkamp, Bernd, Dr.**
**Claudiusweg 5**
**D-5600 Wuppertal 1(DE)**

(54) **Verfahren zur Herstellung von 1-Aryl-5-aminopyrazolen.**

(57) Die Erfindung betrifft ein Verfahren zur Herstellung von 1-Aryl-5-aminopyrazolen der Formel (I)

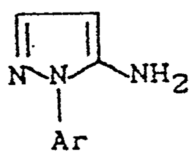

(I)

in welcher
Ar für gegebenenfalls substituiertes Phenyl oder Pyridyl steht,
dadurch gekennzeichnet, daß man Arylhydrazin-Hydrochloridsalze der Formel (II)
$Ar-NH-NH_2 \times HCl$ (II)
in welcher
Ar die oben angegebene Bedeutung hat,
mit dem Formylacetonitril-Natriumsalz der Formel (III)
NaOCH = CH-CN (III)
in Gegenwart eines Lösungsmittels umsetzt und die dabei entstehenden Hydrazone der Formel (IV)
$Ar-NH-N=CH-CH_2-CN$ (IV)
in welcher
Ar die oben angegebene Bedeutung hat,
gegebenenfalls in Gegenwart eines Katalysators bei Temperaturen zwischen 0 °C und 120 °C cyclisiert.

EP 0 242 687 A2

# Verfahren zur Herstellung von 1-Aryl-5-aminopyrazolen

Die Erfindung betrifft ein neues Verfahren zur Herstellung von bekannten 1-Aryl-5-aminopyrazolen, die als Zwischenprodukte für die Synthese von Verbindungen mit herbizider und insektizider Wirksamkeit verwendet werden können.

Es ist bereits bekannt, daß man 1-Aryl-5-aminopyrazole erhält, indem man Arylhydrazine mit 2,3-Dibrompropionitril oder α-Chloracrylnitril umsetzt (vgl. J. Prakt. Chem. 321, 93, (1979); DE-OS 27 01 316 und DD-OS 126 303). Nachteilig an diesem Verfahren sind jedoch der hohe Preis und die schlechte Verfügbarkeit des als Reaktionskomponente benötigten 2,3-Dibrompropionitrils und α-Chloracrylnitrils. Außerdem wirkt sich für die Verfahrensdurchführung insbesondere der während des Reaktionsverlaufs anfallende hohe Salzgehalt im Reaktionsgemisch äußerst nachteilig aus.

Ferner ist bekannt, daß man 1-Aryl-5-aminopyrazole erhält, indem man Arylhydrazine mit Cyanacetylen umsetzt (vgl. Takedo Kenkyusho Ho, 1971, 30, 475 [C.A. 76/85737 (1972)]). Nachteilig an diesem Verfahren sind wiederum der hohe Preis und die Zugänglichkeit des als Reaktionskomponente notwendigen Cyanacetylens welches sich bei Lagerung zersetzt.

Weiterhin ist bekannt, daß man 1-Aryl-5-aminopyrazole durch Umsetzung von Arylhydrazinen mit ß-Dimethylaminoacrylnitril erhält (vgl. Helv. Chim. Acta, 48, 1754 und DE-OS 21 41 700).

Wiederum sind der hohe Preis und die schlechte Verfügbarkeit des ß-Dimethylaminoacrylnitrils von Nachteil.

Außerdem ist bekannt, daß man 1-Aryl-5-aminopyrazole erhält, indem man Isoxazol im alkalischen Medium zu Cyanacetaldehyd umsetzt, anschließend mit Arylhydrazinen im sauren Medium zu den Arylhydrazonen des Cyanacetaldehyds kondensiert und schließlich im alkalischen Medium cyclisiert (vgl. Chem. Ber. 42, 59, (1909)). Der Nachteil dieses Verfahrens besteht in der schlechten Gesamtausbeute und den allgemeinen Nachteilen einer mehrstufigen Reaktionsführung.

Schließlich ist bekannt, daß man 1-Aryl-5-aminopyrazole erhält, indem Malondialdehyddioxim mit salpetriger Säure und Arylhydrazin umsetzt (vgl. Liebigs Ann., 729, 139 (1969) und DE-OS 19 13 845). Nachteilig an diesem Verfahren sind der hohe Preis und die schlechte Verfügbarkeit des als Reaktionskomponente benötigten Malondialdehyddioxims.

Es wurde nun gefunden, daß man bekannte 1-Aryl-5-aminopyrazole der allgemeinen Formel (I)

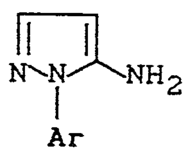 (I)

in welcher

Ar für gegebenenfalls substituiertes Phenyl oder gegebenenfalls substituiertes Pyridyl steht,

erhält, wenn man Arylhydrazin-Hydrochloridsalze der Formel (II)

$Ar\text{-}NH\text{-}NH_2 \times HCl$ (II)

in welcher

Ar die oben angegebene Bedeutung hat,

mit dem Formylacetonitril-Natriumsalz der Formel (III)

$NaOCH=CH\text{-}CN$ (III)

in Gegenwart eines Lösungsmittels umsetzt und die dabei entstehenden Hydrazone der Formel (IV)

$Ar\text{-}NH\text{-}N=CH\text{-}CH_2\text{-}CN$ (IV)

gegebenenfalls nach Zwischenisolierung und gegebenenfalls in Gegenwart eines Katalysators bei Temperaturen zwischen 0 °C und 120 °C cyclisiert.

Es ist als ausgesprochen überraschend zu bezeichnen, daß man mit Hilfe des erfindungsgemäßen Verfahrens die gewünschten 1-Aryl-5-amino-pyrazole in hohen Ausbeuten und guter Reinheit erhält, da aus dem Stand der Technik zu erwarten war, daß das Hydrochlorid mit dem Natriumsalz des Formylacetonitrils das freie Formylacetonitril ergibt, welches besonders im basischem Medium ziemlich unbeständig ist und Selbstkondensation eingeht (vgl. F. Raubin, G. Toupance. Bull. Soc. Chim., France 1975, 188).

Das erfindungsgemäße Verfahren zeichnet sich durch eine Reihe von Vorteilen aus. So ermöglicht es die Herstellung von 1-Aryl-5-aminopyrazolen in hohen Ausbeuten und guter Reinheit, wobei die Ausgangsprodukte gut zugänglich sind. Ferner ist die Umsetzung äußerst einfach und wirtschaftlich in einem Eintopfverfahren durchführbar und die Isolierung der 1-Aryl-5-aminopyrazole bereitet keinerlei Schwierigkeiten.

Mit Hilfe des erfindungsgemäßen Verfahrens erhält man vorzugsweise solche 1-Aryl-5-aminopyrazole der Formel (I),

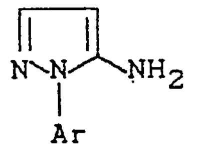

(I)

bei welcher
Ar für einfach oder mehrfach, gleich oder verschieden substituiertes Phenyl oder für jeweils gegebenenfalls einfach oder mehrfach gleich oder verschieden substituiertes 2-Pyridyl, 3-Pyridyl oder 4-Pyridyl steht, wobei als Phenyl-und Pyridylsubstituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R'$,
wobei
R' für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und
p für eine Zahl 0, 1 oder 2 steht.

Das erfindungsgemäße Verfahren betrifft besonders beforzugt Verbindungen der Formel (I), in welcher
Ar für ein-bis fünffach, gleich oder verschieden substituiertes Phenyl oder für jeweils gegebenenfalls ein-bis vierfach gleich oder verschieden substituiertes 2-Pyridyl oder 4-Pyridyl steht, wobei als Phenyl-bzw. Pyridylsubstituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R'$,
wobei
R' für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht und
p für eine Zahl 0, 1 oder 2 steht.

Verwendet man beispielsweise 2,6-Dichlor-4-trifluormethylphenylhydraziniumchlorid und Natrium-Formylacetonitril als Ausgangsstoffe, Ethanol als Lösungsmittel und Natriumhydroxid als Katalysator, so kann der Verlauf des erfindungsgemäßen Verfahrens durch das folgenden Formelschema veranschaulicht werden:

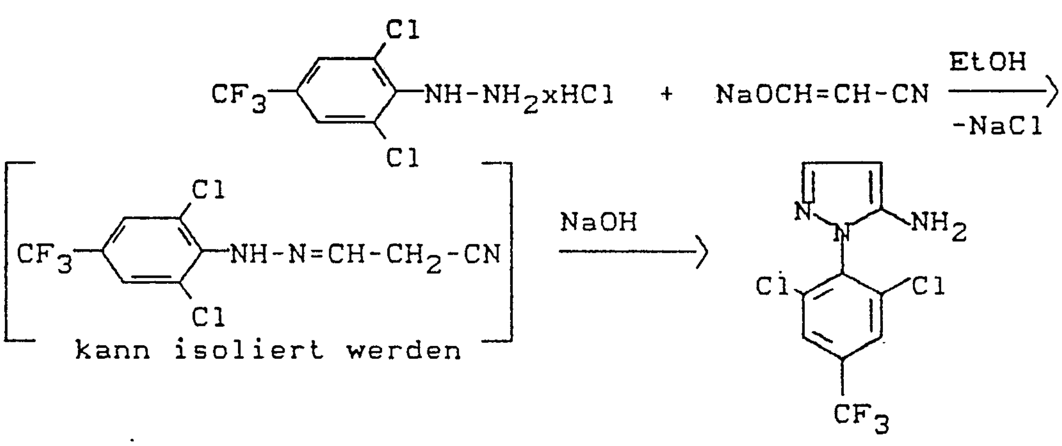

Die zur Durchführung des erfindungsgemäßen Verfahrens als Ausgangsstoffe benötigten Arylhydrazin-Hydrochloridsalze sind durch die Formel (II) allgemein definiert. In dieser Formel (II) steht Ar vorzugsweise für diejenigen Reste, die bereits im Zusammenhang mit der Beschreibung der Endprodukte der Formel (I) als bevorzugt für diesen Substituenten genannt wurden.

Die Arylhydrazin-Hydrochloridsalze der Formel (II) sind bekannt bzw. können nach bekannten Verfahren in analoger Weise hergestellt werden (vgl. z.B. Houben-Weyl "Methoden der organischen Chemie" Band X/2, S. 203, Thieme Verlag Stuttgart 1967 und DE-OS 34 02 308).

Sie können beispielsweise erhalten werden, indem man das entsprechend substituierte Arylhalogenid mit Hydrazinhydrat in einem Lösungsmittel wie z.B. Pyridin unter Rückfluß erhitzt und nach Aufarbeitung und Aufnehmen in Ethanol mit konzentrierter Salzsäure ausfällt.

Das Formylacetonitril-Natriumsalz der Formel (III) ist bekannt (vgl. DE-OS 27 53 322). Es kann beispielsweise erhalten werden, indem man Acetonitril und Kohlenmonoxid in Gegenwart eines Verdünnungsmittels wie z.B. Toluol und in Gegenwart einer Base wie z.B. Natriumethylat umsetzt.

Das erfindungsgemäße Verfahren wird in Gegenwart eines Verdünnungsmittels durchgeführt. Hierzu kommen vorzugsweise Alkohole wie z.B. Methanol, Ethanol, n-und i-Propanol und n-und i-Butanol sowie dipolare aprotische Lösungsmittel wie insbesondere Ether, wie z.B. Tetrahydrofuran, Dioxan Glycoldimethylether und Diethylenglykoldimethylether und Sulfoxide, wie Dimethylsulfoxid, in Frage.

Das erfindungsgemäße Verfahren wird gegebenenfalls in Gegenwart eines Katalysators durchgeführt. Hierzu gehören insbesondere Alkalimetallhydroxide wie z.B. Natriumhydroxid und Kaliumhydroxid sowie Alkalimetallalkoholate wie z.B. Natrium-oder Kaliummethylat und Natrium-oder Kaliumethylat. Vorzugsweise arbeitet man bei dem erfindungsgemäßen Verfahren mit Katalysator.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 120 °C, vorzugsweise bei Temperaturen zwischen 20 °C und 80 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Bei der Durchführung des erfindungsgemäßen Verfahrens setzt man auf 1 Mol Arylhydrazin-Hydrochloridsalz der Formel (II) 1 bis 3 Mol, vorzugsweise 1 bis 1,5 Mol, insbesondere 1 Mol Formylacetonitril-Natriumsalz der Formel (III) und gegenenfalls 0,1 bis 1 Mol, vorzugsweise 0,3 Mol Katalysator ein.

Die Reaktion wird so durchgeführt, daß man die Reaktionspartner in dem entsprechenden Lösungsmittel unter Rückfluß erhitzt.

Man kann aber auch die Reaktion so durchführen, daß man die Reaktionspartner in dem entsprechenden Lösungsmittel für 4 bis 6 Stunden unter Rückfluß erhitzt und anschliessend entweder in Gegenwart der entsprechenden Base bei Temperaturen zwischen 20 und 40 °C für 6 bis 12 Stunden rührt oder das entsprechende Hydrazon zwischenisoliert und dann entweder in Gegenwart der entsprechenden wässrigen Base und gegebenenfalls in Gegenwart eines Emulgators, wie beispielsweise Emulgator A (Polyetherester aus Ölsäure und 6 Mol Ethylenoxid) oder in Gegenwart eines wassermischbaren Lösungsmittels und der entsprechenden wässrigen Base bei Temperaturen zwischen 20 °C und 60 °C für 6 bis 12 Stunden rührt.

Die Isolierung der 1-Aryl-5-aminopyrazole der Formel (I) erfolgt in üblicher Art und Weise, indem man beispielsweise das Reaktionsgemisch im Vakuum einengt, den Rückstand mit einem mit Wasser nicht mischbaren Lösungsmittel extrahiert, die organische Phase mit Wasser wäscht, tocknet und das Lösungsmittel abdestilliert.

Die nach dem erfindungsgemäßen Verfahren herstellbaren 1-Aryl-5-amino-pyrazole der Formel (I) sind bekannte Ausgangsstoffe zur Synthese von biologisch aktiven Verbindungen, wie z.B. zur Synthese von substituierten 5-Amino-1-phenyl-pyrazolen (vgl. DE-OS 34 02 308), welche gute herbizide Eigenschaften besitzen. In entsprechenden Aufwandmengen sind auch die nach dem erfindungsgemäßen Verfahren herstellbaren 1-Aryl-5-aminopyrazole selbst herbizid wirksam (vgl. DE-OS 34 02 308).

So läßt sich beispielsweise das 5-Propionamido-1-(2,6-dichlor-4-trifluormethylphenyl)-pyrazol der Formel

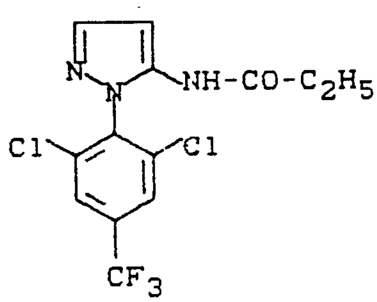

herstellen, indem man 5-Amino-1-(2,6-dichlor-4-trifluormethylphenyl)-pyrazol mit Propionylchlorid in Gegenwart von Dichlormethan und Pyridin umsetzt. Diese Synthese läßt sich formelmäßig wie folgt veranschaulichen:

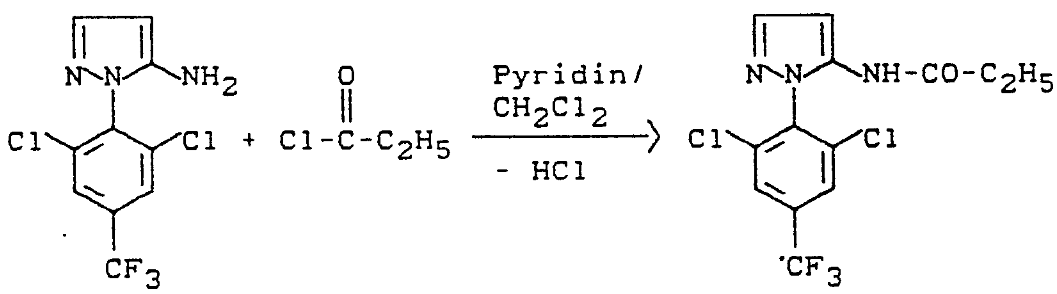


Das erfindungsgemäße Verfahren wird durch die folgenden Beispiele veranschaulicht.

Beispiel 1

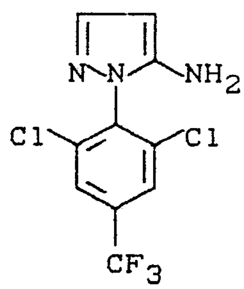

5,6 g (0,02 Mol) 2,6-Dichlor-4-trifluormethylphenylhydraziniumchlorid und 2,0 g (0,022 Mol) Natrium-Formylacetonitril werden in 30 ml Ethanol 6 Stunden unter Rückfluß erhitzt. Anschließend tropft man 0,8 g 40-prozentige Natronlauge in 60 ml Wasser zu und rührt ca. 12 Stunden bei 40 °C nach. Man entfernt den Ethanol im Vakuum, nimmt den Rückstand in Dichlormethan auf, wäscht mit Wasser, trocknet über Natriumsulfat und entfernt das Lösungsmittel im Vakuum.

Man erhält 5,0 g (84,5 % der Thorie) an 1-(2,6-Dichlor-4-trifluormethylphenyl)-5-aminopyrazol vom Schmelzpunkt 90 - 94 °C.

Herstellung der Ausgangsverbindung:

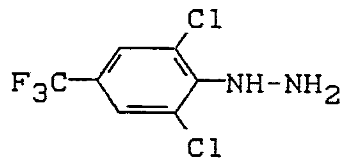

6,2 g (0,025 Mol) 3,4,5-Trichlor-trifluormethylbenzol und 6,25 g (0,125 Mol) Hydrazinhydrat werden in 12 ml Pyridin 48 Stunden bei 115 - 120 °C unter Rückfluß erhitzt. Zur Aufarbeitung destilliert man das Lösungsmittel ab, nimmt den Rückstand in Wasser auf und extrahiert dreimal mit jeweils ca. 30 ml Dichlormethan. Die vereinigten organischen Phasen werden über Magnesiumsulfat getrocknet, im Vakuum eingeengt und anschließend destilliert.

Man erhält 5,1 g (83 % der Theorie) an 2,6-Dichlor-4-trifluormethylphenylhydrazin vom Schmelzpunkt 56 bis 57 °C mit einem gaschromatographisch bestimmten Gehalt von 90 %.

(Verfahrensvariante)

56 g (0,2 Mol) (2,6-Dichlor-4-trifluormethylphenyl)-hydraziniumchlorid und 21,8 g (0,22 Mol) Natrium-Formylacetonitril werden in 300 ml Ethanol 6 Stunden unter Rückfluß erhitzt. Anschließend filtriert man das Reaktionsgemisch und engt das Filtrat ein. Der Rückstand wird in 600 ml Wasser, 8 g 40-prozentiger Natronlauge und 2-3 Tropfen Emulgator A (Polyetherester aus Ölsäure und 6 Mol Ethylenoxid) 14 Stunden bei 40 °C gerührt. Das Reaktionsgemisch wird in Wasser gegossen und mit Methylenchlorid extrahiert. Die organische Phase wird getrocknet, eingeengt und andestilliert.

Man erhält 48,3 g (83,2 % der Theorie) an 1-(2,6-Dichlor-4-trifluormethylphenyl)-5-aminopyrazol vom Schmelzpunkt 90 - 94 °C.

Beispiel 2

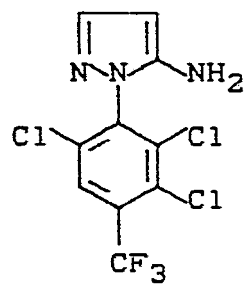

6,32 g (0,02 Mol) 2,3,6-Trichlor-4-trifluormethylphenylhydraziniumchlorid und 2,28 g (0,025 Mol) Natrium-Formylacetonitril werden in 30 ml Ethanol 14 Stunden unter Rückfluß erhitzt. Anschließend gibt man 40 ml Wasser und 0,74 g 45-%ige Natronlauge sowie 1 Tropfen Emulgator A zu und erhitzt weitere 20 Stunden bei 40°C. Man entfernt den Ethanol und extrahiert die wäßrige Phase mit Methylenchlorid. Die organische Phase wird getrocknet, eingeengt und abdestilliert.

Man erhält 5,2 g (79 % der Theorie) an 1-(2,3,6,Trichlor-4-trifluormethylphenyl)-5-amino-pyrazol vom Schmelzpunkt 105 - 108 °C.

Herstellungsbeispiel für eine herbizid wirksame Verbindung

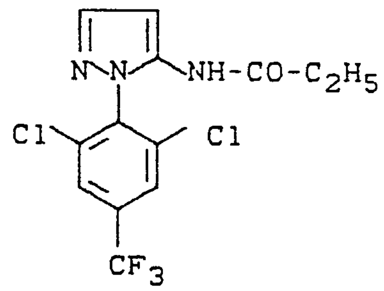

14,8 g (0,05 Mol) 5-Amino-1-(2,6-dichlor-4-trifluormethylphenyl)-pyrazol in 100 ml Dichlormethan werden bei Raumtemperatur unter Rühren nacheinander mit 5 ml (5,3 g / 0,05 Mol) 98%igem Propionylchlorid und dann mit 5 ml (5,0 g / 0,063 Mol) wasserfreiem Pyridin versetzt. Dabei steigt die Temperatur auf 40 °C. Nach beendeter Zugabe rührt man weitere 16 Stunden bei Raumtemperatur, gibt 50 ml Dichlormethan zu, wäscht je zweimal mit 100 ml Wasser, 100 ml gesättigter Natriumhydrogencarbonatlösung und 100 ml Natriumchloridlösung, trocknet über Magnesiumsulfat und entfernt das Lösungsmittel im Vakuum. Der feste Rückstand wird mit wenig Hexan gewaschen und getrocknet.

Man erhält 12,2 g (69,3 % der Theorie) an 5-Propionamido-1-(2,6-dichlor-4-trifluormethylphenyl)-pyrazol vom Schmelzpunkt 125 °C.

## Ansprüche

1. Verfahren zur Herstellung von 1-Aryl-5-aminopyrazolen der Formel (I)

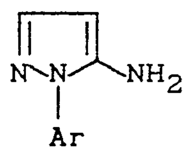

(I)

in welcher
Ar für gegebenenfalls substituiertes Phenyl oder gegebenenfalls substituiertes Pyridyl steht,
dadurch gekennzeichnet, daß man Arylhydrazin-Hydrochloridsalze der Formel (II)
$Ar\text{-}NH\text{-}NH_2 \times HCl$ (II)
in welcher
Ar die oben angegebene Bedeutung hat,
mit dem Formylacetonitril-Natriumsalz der Formel (III)
$NaOCH=CH\text{-}CN$ (III)
in Gegenwart eines Lösungsmittels umsetzt und die dabei entstehenden Hydrazone der Formel (IV)
$Ar\text{-}NH\text{-}N=CH\text{-}CH_2\text{-}CN$ (IV)
in welcher
Ar die oben angegebene Bedeutung hat,
gegebenenfalls in Gegenwart eines Katalysators bei Temperaturen zwischen 0 °C und 120 °C cyclisiert.

2. Verfahren gemäß Anspruch 1 dadurch gekennzeichnet, daß man als Lösungsmittel Alkohole, Ether oder Sulfoxide verwendet.

3. Verfahren gemäß Anspruch 1, dadurch gekennzeichnet, daß man als Katalysator Alkalimetallhydroxide oder Alkalimetallalkoholate verwendet.

4. Verfahren gemäß Anspruch 1, dadurch gekennzeichnet, daß man die Reaktion bei Temperaturen zwischen 20° C und 80° C durchführt.

5. Verfahren nach Anspruch 1, dadurch gekennzeichnet, daß man auf 1 Mol Arylhydrazin-Hydrochloridsalz der Formel (II) 1 bis 3 Mol Formylacetonitril-Natriumsalz der Formel (III) und gegebenenfalls 0,1 bis 1 Mol Katalysator einsetzt.

6. Verfahren nach Anspruch 5, dadurch gekennzeichnet, daß man auf 1 Mol Arylhydrazin-Hydrochloridsalz der Formel (II) 1 bis 1,5 Mol Formylacetonitril-Natriumsalz der Formel (III) und gegebenenfalls 0,3 Mol Katalysator einsetzt.

7. Verfahren nach Anspruch 1, dadurch gekennzeichnet, daß man die Umsetzung als Eintopfverfahren durchführt.

8. Verfahren nach Anspruch 1, dadurch gekennzeichnet, daß man die Hydrazone der Formel (IV) isoliert.

9. Verfahren gemäß den Ansprüchen 1 bis 8, dadurch gekennzeichnet, daß man 1-Aryl-5-amino-pyrazole der Formel (I)

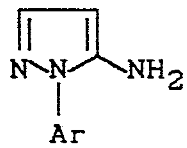

. (I)

erhält, in welcher

Ar für einfach oder mehrfach, gleich oder verschieden substituiertes Phenyl oder für jeweils gegebenenfalls einfach oder mehrfach gleich oder verschieden substituiertes 2-Pyridyl, 3-Pyridyl oder 4-Pyridyl steht, wobei als Phenyl-und Pyridylsubstituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R'$, wobei

R' für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

p für eine Zahl 0, 1 oder 2 steht.